# EUROPÄISCHE PATENTSCHRIFT

(11) **EP 0 832 871 B1**
(45) Veröffentlichungstag und Bekanntmachung des Hinweises auf die Patenterteilung: **20.12.2000**
(21) Anmeldenummer: 97115590.8
(22) Anmeldetag: 09.09.1997
(51) Int. Cl.: C07C 45/67, C07C 49/603

(54) **Verfahren zur Herstellung von Beta-Isophoron durch Isomerisierung von Alpha-Isophoron (I)**
Process for the preparation of beta-isophoron by isomerisation of alpha-isophoron (I)
Procédé pour la préparation de beta-isophorone par isomérisation d'alpha-isophorone

(30) Priorität: 26.09.1996 DE 19639569
(43) Veröffentlichungstag der Anmeldung: 01.04.1998
(73) Patentinhaber: Degussa-Hüls Aktiengesellschaft, 60287 Frankfurt am Main (DE)
(72) Erfinder: Krill, Steffen, Dr., 67346 Speyer (DE); Giray, Günes, Mavischir-Didim (TR); Huthmacher, Klaus, Dr., 63571 Gelnhausen (DE); Hübner, Frank, Dr., 64372 Ober-Ramstadt (DE); Tanner, Herbert, Dr., 63457 Hanau-Wolfgang (DE)

(56) Entgegenhaltungen:
- EP-A- 0 312 735
- EP-A- 0 488 045
- PATENT ABSTRACTS OF JAPAN vol. 013, no. 457 (C-644), 16.Oktober 1989 & JP 01 175954 A (SODA KORYO KK), 12.Juli 1989,

## Beschreibung

Die Erfindung betrifft ein neues Verfahren zur Herstellung von 3,5,5-Trimethylcyclohexa-3-en-1-on (β-Isophoron) durch Isomerisierung von 3,5,5-Trimethylcyclohexa-2-en-1-on (α-Isophoron) in der Flüssigphase in Gegenwart eines homogenen oder heterogenen Katalysators.

β-Isophoron hat großes wirtschaftliches Interesse, da es einen wichtigen Synthesebaustein für die Herstellung von Carotinoiden, Vitaminen und pharmazeutischen Produkten darstellt. Insbesondere wird β-Isophoron als Vorstufe für Ketoisophoron (= 2,6,6-Trimethylcyclohex-2-en-1,4-dion) und Trimethylhydrochinon und damit für die Herstellung von Vitamin E benötigt. Daneben geht es zentral in Synthesen für Riechstoffe und Naturstoffe wie Astaxanthin und Abscisinsäure und Derivate ein.

Die Herstellung von Isophoron gelingt durch Aceton-Trimerisierung, unter Kondensation der C₃-Bausteine. Das hauptsächlich gebildete Isomere ist das α-Isophoron, da es im Gegensatz zum β-Isomeren eine zur Ketofunktion konjugierte Doppelbindung besitzt. Aus diesem Grund liegt das thermodynamische Gleichgewicht auf Seiten des α-Isophorons; die β-Konzentration beträgt nur etwa 1 - 2 % und die Gleichgewichtseinstellung erfolgt sehr langsam.

Obwohl es grundsätzlich zwei verschiedenen Ansätze gibt, zum Ketoisophoron zu gelangen, nämlich die Direktoxidation von α-Isophoron → Ketoisophoron und dem Umweg über die Isomerisierung α-Isophoron → β-Isophoron in einem Primärschritt und anschließende Oxidation des β-Isophoron → Ketoisophoron, ist letzteres Verfahren deutlich vorteilig. Schema 1 stellt diese Überlegungen zur Ketoisophoron-Synthese anschaulich dar:

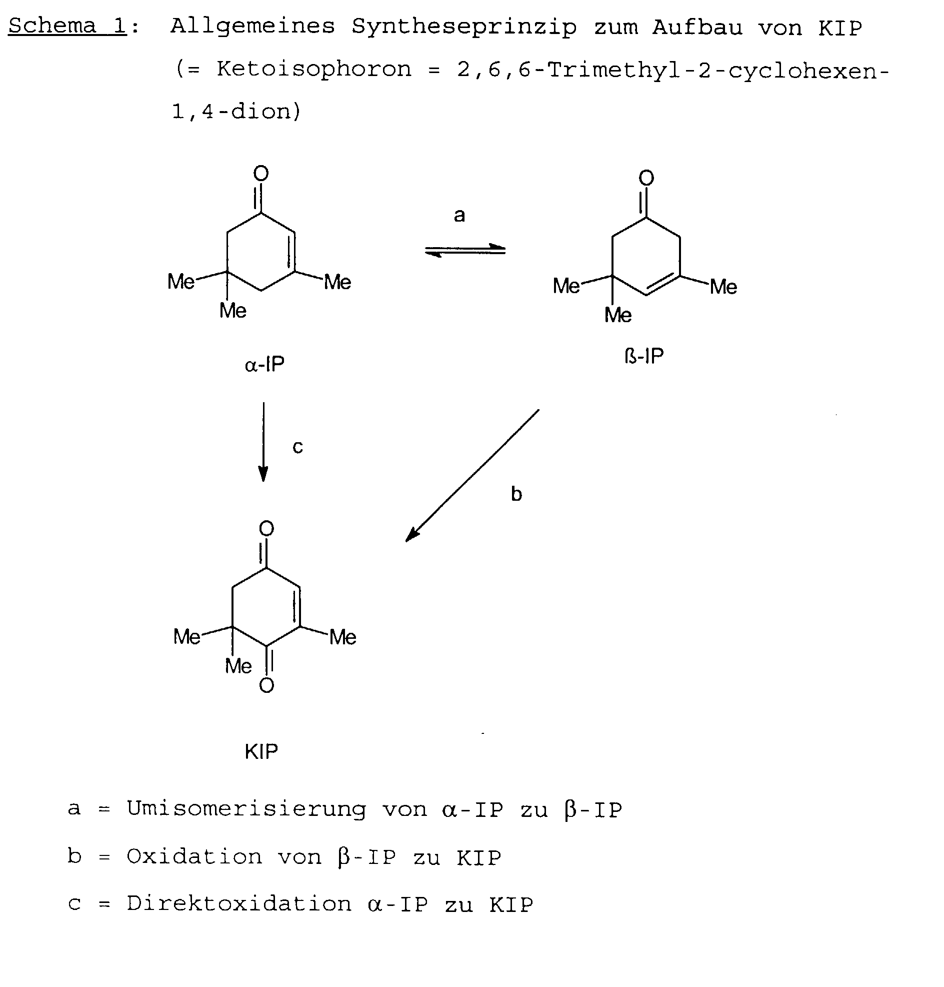

Im Laufe der Zeit sind zahlreiche Verfahren zur Isomerisierung von α-IP beschrieben worden, die jedoch erhebliche Nachteile aufweisen. Gesichtspunkte wie hoher Chemikalienverbrauch, schlechte Raum-/Zeitausbeute und Schwierigkeiten bei der Aufarbeitung haben bisher eine praktische Verfahrensumsetzung im größeren Maßstab verhindert.

Mit der Isomerisierung in der Flüssigphase beschäftigen sich eine Reihe von Druckschriften. Der nähere Stand der Technik wird durch folgende Schriften repräsentiert:
- D1 =: A. Heymes et al., Recherches 1971, 18, 104
- D2: FR-A-1 446 246
- D3 =: DE-OS-24 57157
- D4 =: US-A- 4,005,145
- D5 =: EP-A- 0312 735
- D6 =: JP 87-33019 entspr. HEI-1-175954 v. 12.07.1989

D1 offenbart die Isomerisierung von α-IP zu β-IP mit stöchiometrischen Mengen MeMgX (X = Halogen-) Grignard Verbindung. Man erhält unter Methanfreisetzung in Gegenwart katalytischer Mengen FeCl₃ 73 % β-IP.

D2 betrifft die Umisomerisierung von α-IP zu β-IP in Gegenwart katalytischer Mengen p-Toluolsulfonsäure und allgemein aromatischer Sulfonsäuren, insbesondere Anilinsulfonsäure. Die Einsatzmenge des Katalysators beträgt bezogen auf eingesetztes α-IP 0,1 - 0,2 %. Ein niedriger Umwandlungsgrad und ein hoher Nebenproduktanfall verhindern jedoch eine industrielle Anwendung des Verfahrens der D2.

Gemäß D3 erfolgt die Darstellung von β-IP durch mehrstündiges Kochen von α-IP in Triäthanolamin, Fraktionierung, Waschen des Destillates mit Weinsäure und Kochsalzlösung. Auch hier ist der Chemikalienverbrauch erheblich.

Gemäß D4 werden als Katalysator Säuren mit einem pK = 2 - 5 und einem höheren Siedepunkt als β-IP (Sdpkt. β-IP = 186 °C/760 mm Hg)eingesetzt.Genannt werden:

Aliphatische und aromatische Aminosäuren, Adipinsäure, p-Methyl-Benzoesäure, 4-Nitro-m-Methyl-Benzoesäure, 4-Hydroxy-Benzoesäure, 3,4,5-Trimethoxybenzoesäure, Vanillinsäure, 4-Trifluormethyl-Benzoesäure, 3-Hydroxy-4-Nitrobenzoesäure und Cyclohexancarbonsäure und Derivate. Der Katalysatoreinsatz beträgt 0,1 - 20 Mol %. Die Ausbeute an β-IP (bezogen auf eingesetztes α-IP) beträgt 74,5 %.

Bei einer Abnahmegeschwindigkeit von 11 ml/h β-IP und einer gleichzeitigen Einsatzmenge von etwa 0,5 kg α-IP beläuft sich die Raum-Zeit-Ausbeute und die β-IP-Entstehung mit Y = 0,218 kg β-IP/kg_{Kat}·h und ist damit zu niedrig, um technische Anwendung zu finden. Diese Werte entsprechen einer Raum-Zeit Ausbeute von Y_{R-Z} = 0,015 l_{β-IP}/h/l_{Lösung .}

Nach einem ähnlichen Prinzip geht man in D5 vor. Als π-Bindungsverschiebungskatalysatoren finden Acetylacetonate von Übergangsmetallen Verwendung. Auch Al(acac) zeigt katalytische Aktivität. Der Einsatz des Katalysators erfolgt in 0,01 - 10 wt %. Patentiert sind Metallkatalysatoren der Gruppen IVb (Ti/Zr/Hf), Vb (V/Nb/Ta), VIb (Cr, Mo, W), VIIb (Mn/ Tc/Re) der gesamten Gruppe VIII und Aluminium. Das primär anfallende Destillat hat einen β-IP-Gehalt von 94 %, eine weitere Vigreuxdestillation reichert den β-IP-Gehalt auf 99 % an. Dieses Ergebnis entspricht bezogen auf Katalysatoreinsatzmenge und Zeit einer Ausbeute von Y = 9,4 Liter β-IP pro Kilogramm Katalysator und Stunde.Bezogen auf die eingesetzte Eduktlösung entspricht dies einer Ausbeute von Y_{R-Z} = 0,0376 l_{β-IP}/h/l_{Lösung}.

Gemäß D6 erfolgt die Isomerisierung in der Flüssigphase bei Temperaturen um 200 °C. Als Katalysator findet Kieselgel mit oder ohne Zusatz von alkylsubstituierten Imidazolinen der nachfolgenden Formel Verwendung, 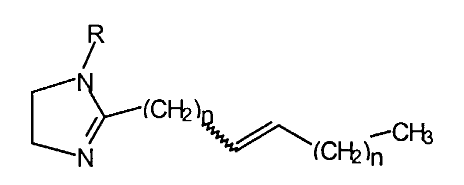 wobei R gleich β-Hydroxyethyl, β-Hydroxypropyl oder γ-Hydroxypropyl ist; m gleich 5-7 und n gleich 0-12 ist.

Typische Experimentbedingungen: 300 g α-IP und 25,7 g SiO₂ werden 52 h in Gegenwart von Edelstahl destilliert, es resultieren 230 g β-IP (= 76,6 % Ausbeute) mit 99,9 % Reinheit. Dieses Ergebnis entspricht bezogen auf Katalysatoreinsatzmenge und Zeit einer Ausbeute von Y = 0,174 Liter β-IP pro Liter Katalysator und Stunde.

Die beschriebene Verfahrensweise ist zudem ungünstig, die absolute β-IP-Entstehung gering. Besonders nachteilig wirkt sich die Durchführung von Isomerisierung und Reindestillation des β-IP in einem Schritt aus. Durch die hohe Reaktions-temperatur in der Destillationsapparatur tritt nachweislich im erheblichen Maße die Rückisomerisierung von β-IP zu α-IP ein.

Angesichts des hier zitierten und diskutierten Standes der Technik war es eine Aufgabe der Erfindung, die oben erwähnten Nachteile der bisherigen Methoden zu vermeiden und ein Verfahren anzubieten, nach dem auf technisch vorteilhafte Weise 3,5,5-Trimethylcyclohexa-3-en-1-on aus seinem Isomeren 3,5,5-Trimethylcyclohexa-2-en-1-on hergestellt werden kann.

Das erfindungsgemäße Verfahren ermöglicht einen hohen Umsatz und übertrifft die bislang aus dem Stand der Technik bekannten Verfahren. Weiterhin werden durch die neue Verfahrensführung sowohl der Anfall von Nebenprodukten verringert als auch die Raum-Zeit-Ausbeute bezogen auf das Volumen der eingesetzten Eduktlösung verbessert.

Die vorliegende Erfindung betrifft ein Verfahren, in dem α-Isophoron zu seinem Isomeren β-Isophoron umgesetzt wird, wobei ein homogener oder ein heterogener Katalysator in der Flüssigphase verwendet wird.

Folgt man dem Stand der Technik, ist es notwendig, ein möglichst reines β-Isophoron aus dem Reaktionsgefäß durch Destillation abzutrennen.
Unter den geläufigen Reaktionsbedingungen der Isomerisierung tritt jedoch bei der Siedetemperatur von 186 ° C teilweise die Rückisomerisierung ein, so daß die Raum-Zeit-Ausbeute in Bezug auf β-Isophoron stark beeinträchtigt wird.

Es wurde nun gefunden, daß es entgegen dieser allgemeinen Auffassung vorteilhafter ist, aus dem Reaktionsgefäß, in dem die Isomerisierung stattfindet, ein Gemisch destillativ abzuziehen, das 0,5 bis 75 Gew.-%, insbesondere 0,5 bis 60 Gew.-%, insbesondere 0,5 bis 40 Gew.-% an β-Isophoron enthält.

Als wesentlich für die optimale Auslegung der Abtrennungsrate stellt sich die Abstimmung zwischen der entstehenden Menge an β-Isophoron und der abdestillierten Menge dar.

Vorteilhaft entspricht die Abnahmegeschwindigkeit am Kopf des Reaktionsgefäßes in etwa der Bildungsgeschwindigkeit von β-Isophoron im Reaktionsgefäß.

Das Verfahren gemäß Erfindung wird über einen Temperaturbereich zwischen 100 und <300 °C ausgeführt. Bevorzugt ist der Temperaturbereich zwischen 150 und 260 °C.

Der Zusatz eines Verdünnungs-oder Lösungsmittels ist zwar möglich, aber nicht erforderlich.

Vorzugsweise wird die Reaktion bei einem Druck von 1000 bis 3 x 10⁵ Pa, insbesondere bis 1,5 x 10⁵ Pa Überdruck durchgeführt. Ganz besonders günstige Isomerisierungsparameter sind ∼ 10⁴ Pa bis Normaldruck (etwa 1 x 10⁵ Pa) in Kombination mit der Siedetemperatur des α-Isophorons.

Das erfindungsgemäße Verfahren läßt sich hervorragend kontinuierlich betreiben. Die das Isomerisat enthaltende Flüssigphase wird nach deren Abtrennung zur Trennung von α-Isophoron und β-Isophoron unter Vakuum bevorzugt bei einem Druck von 100 bis 3 x 10⁴ Pa destilliert. Das β-Isophoron fällt dann mit einer Reinheit > 97 % an.

Die Destillation findet im Gegensatz zum Stand der Technik bei Temperaturen statt, bei denen die thermisch bedingte Rückisomerisierung weitgehend ausgeschlossen ist.

Dabei erweist es sich als vorteilhaft, das Sumpfprodukt der Destillation in die Isomerisierungsstufe zu recyclieren.

Durch dieses, dem Stand der Technik widersprechenden Vorgehen, bei dem man ein möglichst hochprozentiges β-Isophoron aus dem Reaktionsgefäß abzieht, erzielt man sowohl eine auf die Eduktlösung bezogene höhere Raum-Zeit-Ausbeute als auch eine auf die Katalysatormenge höhere Ausbeute an β-Isophoron pro kg des eingesetzten Katalysators.

Als solche sind alle aus dem Stand der Technik bekannten heterogenen Katalysatoren geeignet.

Dabei kann die Isomerisierung gegebenenfalls auch in Gegenwart einer organischen Base durchgeführt werden.

Als heterogene Katalysatoren im Sinne der Erfindung werden beispielsweise Oxide oder Mischoxide eines Elements der Gruppen IIₐ, VIII, I_{b}, IIIₐ und Vₐ des Periodensystems der Elemente oder auch unter Versuchsbedingungen unlösliche Salze der genannten Elemente, wie insbesondere Carbonate oder Halogenide ebenso wie SiO₂ eingesetzt. Die Gruppeneinteilung der Haupt- und Nebengruppen des Periodensystems der Elemente erfolgt nach der Bezeichnung gemäß IUPAC, Pure and Appl. Chem.66, 2423-2444, 1994. So gehören zur Gruppe IIa die Metalle Be, Mg, Ca, Sr und Ba. Zur Gruppe VIII die Metalle Fe, Co, Ni, Ru, Rh, Pd, Os, Ir und Pt. Zu den Gruppen I_{b}, IIIₐ und Vₐ die Elemente Cu, Ag, Au, B, Al, Ga, In, Tl, N, P, As, Sb und Bi.

Zu den erfindungsgemäß als heterogene Katalysatoren einsetzbaren Verbindungen gehören die Oxide oder Mischoxide der vorgenannten Elemente. Hierbei wird unter Mischoxid eine Verbindung verstanden, in der Sauerstoff mit mehr als einem der aufgezählten Elemente eine Verbindung eingeht.

Zu den im Rahmen der Erfindung einsetzbaren Oxiden gehören BeO, MgO, CaO, SrO, BaO, TiO₂, ZrO₂, MoO₃, Fe₃O₄, Fe₂O₃, CoO, Co₃O₄, NiO, PdO₂, PtO₂, ZnO, Al₂O_{3,}SiO_{2,} Silikagel .

Zu den im Rahmen der Erfindung einsetzbaren Mischoxiden gehören neben Mischverbindungen oben aufgezählter Oxide insbesondere Al₂O₃/SiO₂ und Zeolithe verschiedener Module, z.B.H-ZSM-5.

Von den obengenannten Oxiden oder Mischoxiden sind insbesondere diejenigen bevorzugt, welche ein Element der Gruppen II_{a,}IIIₐ oder VIII des Periodensystems oder SiO₂ enthalten.

Ganz besonders bevorzugt werden im Rahmen der Erfindung Oxide oder Mischoxide des Calciums und/oder Magnesiums verwendet.

In einer weiteren Ausgestaltung des erfindungsgemäßen Verfahrens ist es bevorzugt, daß ein Oxid oder Mischoxid des Kobalt und/oder Nickel verwendet wird.

Ganz besonders bevorzugte Oxide sind u. a. Co₃O₄ sowie MgO und CaO.

Ein weiterhin besonders bevorzugter Katalysator ist γ -Al₂O₃.
Besonders geeignet sind Cobalt- und Nickelcarbonate, gegebenenfalls in ihrer Hydratform.

Neben der Verwendung von Oxiden oder Mischoxiden als erfindungsgemäße heterogene Katalysatoren zur Isomerisierung von α-Isophoron zu β-Isophoron können mit gutem Erfolg auch mit elementaren Metallen dotierte Oxide und Mischoxide der Gruppen IIₐ, VIII, I_{b}, IIIₐ und Vₐ des Periodensystems der Elemente sowie SiO₂ eingesetzt werden. Zur Dotierung können Elemente, insbesondere Metalle, aus denselben Gruppen des Periodensystems verwendet werden. Zu den vorzugsweise einzusetzenden Dotierungsmetallen gehören u. a. die Metalle der Gruppen VIII sowie IIₐ. In besonderer Abwandlung kennzeichnet sich das erfindungsgemäße Verfahren dadurch, daß ein Katalysator eingesetzt wird, der mit einem Metall aus der Gruppe VIII des Periodensystems dotiert ist. Innerhalb der Gruppe VIII wiederum sind die Metalle Kobalt und/oder Nickel als Dotierungsmetalle besonders günstig.

Die Menge des zur Dotierung eingesetzten Metalles ist an sich nicht besonders kritisch und kann daher über einen weiten Bereich variiert werden. Bevorzugt ist es, daß das Dotierungsmetall in einer Menge von 0,1 bis 60 Gew.-% (wt/wt) bezogen auf das Oxid oder Mischoxid eingesetzt wird. Ein besonders wirksamer Katalysator wird erhalten, wenn ein mit Nickel und/oder Kobalt dotiertes γ-Al₂O₃ oder Co₃O₄ eingesetzt wird.

Ferner kann der Katalysator bzw. auch der mit einem Metall dotierte Katalysator in reiner Form vorliegen oder auf einem Trägermaterial fixiert oder mit dem Träger gemischt sein, wobei das Trägermaterial einer der beschriebenen Katalysatoren sein kann. Weitere Trägermaterialien sind dem Fachmann bekannt. Hierzu gehören Träger wie α-Al₂O₃, γ-Al₂O₃,SiO_{2,} SiO₂/Al₂O₃ verschiedener Module, Aluminiumsalze wie etwa Aluminiumsilikate und Aluminiumphosphate, Aktivkohle etc.

Auch die Menge des zur Isomerisierung einzusetzenden Katalysators kann grundsätzlich über einen größeren Bereich variiert werden. Bevorzugt ist es hierbei, daß der Katalysator in einem Verhältnis von zwischen 0,01 und 50 Gew.-% (wt/wt) bezogen auf α-Isophoron eingesetzt wird. In einer besonders bevorzugten Ausführungsvariante kennzeichnet sich das Verfahren der Erfindung dadurch, daß der Katalysator in einem Verhältnis von zwischen 0,2 und 10 Gew.-% (wt/wt) bezogen auf α-Isophoron eingesetzt wird.

In noch einer weiteren besonders bevorzugten Ausführungsform liegt das Verhältnis von Katalysator zu α -Isophoron im Bereich zwischen 0,5 und 5 Gew.-% (wt/wt).

Das erfindungsgemäße Verfahren ist jedoch nicht auf die heterogen katalysierte Reaktion beschränkt.
Auch die homogen katalysierte Isomerisierung wird durch das erfindungsgemäße Vorgehen erfaßt.

Die Bedingungen für die Umsetzung als solche und die dafür eingesetzten homogenen Katalysatoren sind aus dem Stand der Technik bekannt.

Explizit sind noch einmal zu nennen die
- Acetylacetonate von Übergangsmetallen (IUPAC-Klassifizierung) der Gruppen IV_{b}, V_{b}, VI_{b}, VII_{b} und VIII sowie von Aluminium (aus der DE-OS 3735211 = EP-A - 0312 735).
- Brönstedtsäuren, die unter den thermischen Reaktionsbedingungen stabil sind und einen pK-Wert von 2 - 5 besitzen. Hierzu gehören beispielsweise p-Toluolsulfonsäuren und verschiedene substituierte Derivate (s. belg. Patent 826 113), allgemein aromatische Sulfonsäure.
- Organische Säuren mit einem pK von 2-5 (aus US-PS 4,005,145):
- monocyclisch, aromatisch, oder alicyclisch substituierte, mono-, di- oder oligo-Carbonsäuren, z. B. Adipinsäure, p-Methyl-Benzoesäure, 4-Nitro-m-Methyl-Benzoesäure,< 4-Hydroxy-Benzoesäure, 3,4,5-Trimethoxybenzoesäure, Vanillinsäure, 4-Trifluormethyl-Benoesäure, 3-Hydroxy-4-Nitrobenzoesäure und Cyclohexancarbonsäure und Derivate.

Der homogene Katalysator wird bevorzugt in einem Gewichtsanteil von 0,1 bis 5, insbesondere 0,1 bis 1 Gew.-%, bezogen auf das α-Isophoron, eingesetzt.

### Beispiel 1:

Es wird käufliches Co₃O₄ der Firma Merck (Co(II/III)oxid) verwendet. Die Katalysatoreinsatzform ist in diesem Fall pulverförmig, jedoch ist auch eine granulierte Form katalytisch aktiv. Eine Vorbehandlung des Katalysators ist nicht notwendig. Die Apparatur zur Durchführung der Isomerisierung besteht aus einem Umlauferhitzer, der mit zwei elektrisch versorgten Stabeinsätzen beheizt wird. Man legt 700 ml technisches α-Isophoron vor (Firma Atochem > 98 %) und gibt 25 g Co₃O₄ hinzu. Über dem Umlauferhitzer sitzt eine Destillationskolonne mit 1,2 m Länge und 25 mm Innendurchmesser, die mit V4A Raschigringen von 4 mm ⌀ gefüllt ist. Man erhitzt die Suspension bei Normaldruck bis zur Siedetemperatur und stimmt die Mengen des über eine Telabpumpe zugeförderten α-IP und des abgenommenen Destillats aufeinander ab. In Abhängigkeit von der Abnahmegeschwindigkeit stellt sich folgender β-IP-Gehalt im Primärdestillat ein.

| **Abnahmegeschwindigkeit** | **20 ml/h** | **40 ml/h** | **80 ml/h** | **120 ml/h** | **160 ml/h** | **260 ml/h** |
|---|---|---|---|---|---|---|
| Gehalt β-IP [%] | 47,3 | 44,4 | 38,9 | 33,6 | 26,8 | 19,0 |
| Entstehung β-IP/h [ml/h] | 9,4 | 17,7 | 31,1 | 40,4 | 43,5 | 49,5 |

Die Sumpftemperatur der Isomerisierung bliebt während der Reaktionsdauer konstant bei 216-217 °C. Das anfallende Primärdestillat wird auf eine Destillationskolonne gefördert, die bei einem Unterdruck von 5 mbar - 100 mbar arbeitet. Das bei 12 mbar anfallende Kopfprodukt hat einen Siedepunkt von 55 - 58 °C und besteht zu > 97 % aus β-Isophoron. Bei der beschriebenen Dimensionierung des Versuchs entstehen pro Stunde 50 g β-Isophoron. Das als Sumpfprodukt nicht umge-setzte α-Isophoron wird mit einem Rest-β-Gehalt < 3 % auf die Isomerisierungseinheit zurückgefahren. Die Selektivität bezogen auf den Umsatz beträgt > 98 %. Die Ausbeute bezogen auf die eingesetzte Katalysatormenge beträgt Y=1,98 l_{β-IP}/h/kg_{Kat.} . Die Raum-Zeit Ausbeute bezogen auf das Volumen der zu isomerisierenden Lösung beträgt Y_{R-Z} = 0,0707 l_{β-IP}/h/l_{Lösung}.

### Beispiel 2

25 g eines Magnesiumoxidkatalysators werden in die bereits beschriebene Apparatur eingefüllt. Bei gleicher kontinuierlicher Fahrweise (siehe Beispiel 1) wird am Kopf der Isomerisierungseinheit je nach Abnahmegeschwindigkeit ein Primärdestillat mit folgender Zusammensetzung abgenommen:

| **Abnahmegeschwindigkeit** | **40 ml/h** | **120 ml/h** | **240 ml/h** |
|---|---|---|---|
| Gehalt β-IP [ml] | 46,0 | 34,8 | 21,5 |
| Entstehung β-IP/h [ml/h] | 18,4 | 41,8 | 51,6 |

Durch weitere Erhöhung der Abnahmegeschwindigkeit kann die absolut entstehende β-IP-Menge optimiert werden.Die Sumpftemperatur der Isomerisierung beträgt über die Dauer der Reaktion konstant 216-217 °C. Die Ausbeute bezogen auf die eingesetzte Katalysatormenge beträgt Y=2,064 l_{β-} _{IP}/h/kg_{Kat.} .Die Raum-Zeit Ausbeute bezogen auf die eingesetzte Eduktlösung beträgt Y_{R-Z} = 0,0737 l_{β-} _{IP}/h/l_{Lösung}.

### Beispiel 3:

In der beschriebenen Apparatur (gleiche Dimensionierung der Isomerisierung wie Beispiel 1) werden 1,160 l α-IP an 4,4 g Co₃O₄-Katalysator umgesetzt (Kobaltschwarz Co₃O₄; MW = 240,8 g/mol; 4,4 g = 18,3 mmol) (IP = 138,21 g/mol; 1,160 ml = 8,393 mmol). In Abhängigkeit der Abnahmegeschwindigkeit erhält man Primärdestillate folgenden β-IP-Gehalts:

| **Abnahmegeschwindigkeit** | **20 ml/h** | **40 ml/h** | **80 ml/h** | **160 ml/h** | **240 ml/h** | **280 ml/h** |
|---|---|---|---|---|---|---|
| Gehalt β-IP [ml] | 52,6 | 47,3 | 40,4 | 25,9 | 16,6 | 14,7 |
| Entstehung β-IP/h [ml/h] | 10,5 | 18,9 | 32,2 | 41,4 | 39,8 | 41,2 |

Die Sumpftemperatur der Isomerisierungseinheit beträgt konstant 216-217 °C. Die Ausbeute bezogen auf die eingesetzte Katalysatormenge beträgt Y=9,363 l_{β-} _{IP}/h/kg_{Kat.}.Die Raum-Zeit Ausbeute bezogen auf die eingesetzte Eduktlösung beträgt Y_{R-Z} = 0,0588 l_{β-} _{IP}/h/l_{Lösung}.

### Beispiel 4:

Anstelle des Kobaltoxid-Katalysators aus Beispiel 3 wird α-Aluminiumoxid (Firma Hoffmann La Roche A2) in der Isomerisierungseinheit eingesetzt. Die Reaktion wird analog Beispiel 1 durchgeführt. Man erhält bei kontinuierlicher Abnahme Gemische α-IP/β-IP der folgenden Zusammensetzung:

| **Abnahmegeschwindigkeit** | **20 ml/h** | **40 ml/h** | **95 ml/h** | **160 ml/h** | **180 ml/h** |
|---|---|---|---|---|---|
| Gehalt β-IP [ml] | 58,4 | 34,5 | 17,6 | 12,2 | 9,7 |
| Entstehung β-IP/h [ml/h] | 11,7 | 13,8 | 16,7 | 19,5 | 17,5 |

Die Isomerisierung wird bei einer konstanten Sumpftemperatur von 216-217 °C durchgeführt. Die Ausbeute bezogen auf die eingesetzte Katalysatormenge beträgt Y=0,78 l_{β-IP}/h/kg_{Kat.} .Die Raum-Zeit Ausbeute bezogen auf die eingesetzte Eduktlösung beträgt Y_{R-Z} = 0,0278 l_{β- IP}/h/l_{Lösung}.

### Beispiel 5:

Als Katalysator wird Co₃O₄ (Firma Merck) ohne Vorbehandlung eingesetzt. Die Dimensionierung der Apparatur entspricht den vorherigen Beispielen, die stöchiometrischen Verhältnisse Katalysator / α-IP entsprechen den Bedingungen von Beispiel 1. Man variiert Druck und Temperatur der Isomerisierungseinheit und untersucht bei konstanter Abnahmegeschwindigkeit von 120 ml/h den β-IP-Gehalt des am Kopf der Kolonne anfallenden Primärdestillats. Die zu den entsprechenden Drücken zugeordneten Isomerisierungstemperaturen sind der Tabelle zu entnehmen.

| **Temperatur Sumpf [°C]** | **Druck [mbar]** | **Gehalt β-IP [FL.%]** |
|---|---|---|
| 216 | 1000 | 33,8 |
| 203 | 770 | 25,8 |
| 192 | 580 | 18,6 |
| 178 | 390 | 14,6 |

### Beispiel 6:

Man betreibt die in den Beispielen 1 - 5 beschriebenen Apparate halbkontinuierlich (nicht umgesetztes α-IP wird nicht erneut mit dem Katalysator in Kontakt gebracht) und befüllt die Isomerisierungseinheit mit 25 g Co₃O₄ (Firma Merck). Dann dosiert man mit einer Telab-Laborpumpe kontinuierlich insgesamt 11 l technisches α-IP zu, wobei ein etwa 20-Fl.%-β-IP/α-IP-Gemisch als Primärdestillat anfällt. Die Sumpftemperatur der Isomerisierungseinheit bleibt während der Reaktionszeit bei konstant 216-217 °C. Die Abnahmegeschwindigkeit beträgt ∼ 250 ml/h, was einer β-IP-Entstehung von 50 [ml β-IP/h] entspricht. Als Sumpfprodukt verbleiben 905 g eines dünnen Öls, das zu 117 g (12,9 %) aus Überkondensaten besteht und zu 87,1 % aus wiedergewinnbarem α-IP. Damit beträgt der Nebenproduktanfall bezogen auf umgesetztes α-IP 5,3 %.

### Beispiel 7:

Man betreibt die in den Beispielen 1 - 5 beschriebenen Apparate kontinuierlich. Die Isomerisierungseinheit ist über eine Telabpumpe mit der Destillationskolonne verbunden. Das im Sumpf der Destillationseinheit anfallende α-IP wird über einen Überlaufbehälter abgenommen und wieder der Isomerisierung zugeführt. Am Kopf der Destillationskolonne wird ein β-Isophoron mit einer Reinheit > 97 % abgenommen. Auf diese Weise werden 3,7 l α-IP (Atochem: > 98 % GC) umgesetzt. Als Katalysator werden 25 g Kobaltschwarz (Fa. Merck) eingesetzt, die Abnahmegeschwindigkeit beträgt 240 - 250 ml/h, die Isomerisierungstemperatur beträgt 216-217 °C. Das Primärgemisch hat einen β-IP-Gehalt von 20 - 22 %. Während der Reaktionsdauer zeigt der Katalysator keine Alterung und kann am Schluß der Reaktion durch Filtration fast vollständig zurückgewonnen werden (23,3 g Co₃O₄). Nach Abschluß der Reaktion verbleiben im Umlaufverdampfer 555 g α-IP und 60 g Hochsieder, die problemlos destillativ getrennt werden können. Als Destillat erhält man 3,07 kg β-IP (Reinheit ∼ 98 %). Die Ausbeute bezogen auf den Umsatz beträgt also 97,6 %, der Nebenproduktanfall beträgt 1,9 %. Der Rest besteht aus Wasser, das durch α-IP-Dimerisierung (= Kondensation) entsteht, bzw. durch das technische Edukt in die Reaktion gelangt.

### Beispiel 8

In einen 2-l-Dreihalskolben mit KPG-Rührer und aufgesetzer 120-cm-Vigreux-Kolonne werden 50 g CaO als Katalysator gegeben und 1,5 l α-Isophoron vorgelegt. Man erniedrigt den Druck der Apparatur auf 350 mbar, wobei die Flüssigkeit bei einer Innentemperatur von 175 - 180 C zu sieden beginnt. Der Dreihalskolben ist zusätzlich mit einem Tropfrichter ausgestattet, der eine kontinuierliche Zugabe von α-IP erlaubt. Die Zugabe von Frisch-α-IP entspricht der Menge an am Kopf der Vigreuxkolonne abgenommener α-IP/β-IP-Mischung. Man nimmt kontinuierlich 200 ml Isomerengemisch ab, deren β-Gehalt etwa 21 - 22 Gew.-% beträgt. Die entstehende Mischung wird im Vakuum destilliert, das im Sumpf der Reindestillation anfallende α-IP wird wieder zum Katalysator zudosiert. Am Kopf der Reindestillation kann β-IP-Produkt mit einer Reinheit >98 % abgenommen werden. Mit dieser Verfahrensweise werden 3 kg α-IP umgesetzt und man erhält 2.850 g eines >98 %-β-IP-Produktes. Die Selektivität, bezogen auf umgesetztes α-IP, beträgt >95 %. Der Katalysator ist nach Regenerierung durch Filtration und Waschen mit α-IP noch aktiv und kann für einen weitere Zyklus eingesetzt werden. Die Ausbeute bezogen auf die eingesetzte Katalysatormenge beträgt Y=0,88 l_{β-IP}/h/kg_{Kat.} .Die Raum-Zeit Ausbeute bezogen auf die eingesetzte Eduktlösung beträgt Y_{R-Z} = 0,0293 l_{β- IP}/h/l_{Lösung}.

### Beispiel 9

Man betreibt in den Beispielen 1 bis 6 beschriebenen Apparaturen diskontinuierlich. Als Katalysator werden 25 g Kieselgel 60 (Merk 7734) eingesetzt, die Isomerisierungsblase wird mit 300 g (=325 ml)α-Isophoron befüllt. Am Kopf der Isomerisierungseinheit fällt in Abhängigkeit von der Abnahmegeschwindigkeit ein α-IP/β-IP-Gemisch folgender Zusammensetzung an:

| Abnahmegeschwindigkeit | 80 ml/h | 120 ml/h | 220 ml/h |
|---|---|---|---|
| Gehalt β-JP [ml] | 37,5 | 22,8 | 13,1 |
| Entstehung β-JP/h [ml/h] | 30 | 27,36 | 28,8 |

Man führt die Reaktion bei Normaldruck und Temperaturen von 216-217 °C Sumpftemperatur durch. Bei der oben dargestellten Versuchsanordnung beträgt die Ausbeute bezogen auf die eingesetzte Katalysatormenge Y=1,2 l_{β-} _{IP}/h/kg_{Kat.} .Die Raum-Zeit Ausbeute bezogen auf die eingesetzte Eduktlösung beträgt Y_{R-Z} = 0,0923 l_{β- IP}/h/l_{Lösung}.
Verfährt man wie in der japanischen Offenlegungsschrift (A) HEI 1-175954 beschrieben (300 g α-IP; 25,7g SiO₂, Abnahmegeschwindigkeit 5g/h) und zieht ein 89% β-IP/α-IP Gemisch/h unter Benutzung von SiO₂ als Katalysator ab, so beträgt die auf die Katalysatormenge bezogene Ausbeute Y=0,174 l_{β-IP}/h/kg_{Kat.} .Die Raum-Zeit Ausbeute bezogen auf die eingesetzte Eduktlösung beträgt Y_{R-Z} = 0,0149 l_{β-} _{IP}/h/l_{Lösung}.

### Beispiel 10

Man benutzt die gleiche Apparatur wie in Beispiel 9 beschrieben und verwendet 5 wt% CoCO₃ (=Kobaltcarbonat, Fa. AMG Kokkola) als Katalysator. Bei einer Abnahmegeschwindigkeit von 25 vol% der eingesetzten α-IP Mischung beträgt die β-IP Bildungsrate 67 g/h/l. Durch Quantifizierung des entstehenden Hochsiederanteils wird eine Selektivität von S = 98 % bestimmt.

## Patentansprüche

1. Verfahren zur Herstellung von 3,5,5-Trimethylcyclohexa3-en-1-on (β-Isophoron) durch die katalysierte Isomerisierung von 3,5,5-Trimethylcyclohexa-2-en-1-on (α-Isophoron) in der Flüssigphase,
dadurch gekennzeichnet,daß man aus dem Reaktionsgemisch während der Isomerisierung destillativ ein Gemisch abzieht, das zu 0,5 bis 75 Gew.-% aus β-Isophoron besteht und aus diesem das β-Isophoron durch Vakuumdestillation isoliert (Reindestillation).

2. Verfahren gemäß Anspruch 1,
dadurch gekennzeichnet, daß die Isomerisierung heterogen katalysiert abläuft.

3. Verfahren gemäß Anspruch 1,
dadurch gekennzeichnet, daß die Isomerisierung homogen katalysiert abläuft.

4. Verfahren gemäß den Ansprüchen 1 bis 3,
dadurch gekennzeichnet, daß das Gemisch zu 0,5 bis 60 Gew.-% aus β-Isophoron besteht.

5. Verfahren gemäß den Ansprüchen 1 bis 3,
dadurch gekennzeichnet, daß das Gemisch zu 0,5 bis 40 Gew.-% aus β-Isophoron besteht.

6. Verfahren gemäß den Ansprüchen 1 bis 5,
dadurch gekennzeichnet, daß man den Sumpf aus der Reindestillation in die Isomerisierungsstufe zurückführt.

7. Verfahren gemäß den Ansprüchen 1 bis 6,
dadurch gekennzeichnet, daß man das Verfahren kontinuierlich durchführt.

8. Verfahren gemäß den Ansprüchen 1, 2, 4 bis 7,
dadurch gekennzeichnet, daß man als Katalysator ein Oxid oder Mischoxid eines Elements der Gruppen IIₐ , VIII, I_{b}, IIIₐ und Vₐ des Periodensystems oder unter Versuchsbedingungen unlösliche Salze der genannnten Elemente oder SiO₂ oder Silikagel verwendet und die Isomerisierung gegebenenfalls unter Zusatz einer organischen Base ausgeführt wird.

9. Verfahren nach den Ansprüchen 1, 2, 4 bis 7,,
dadurch gekennzeichnet, daß ein Oxid oder Mischoxid eines Elements der Gruppen IIₐ und VIII des Periodensystems verwendet wird.

10. Verfahren nach Anspruch 9,
dadurch gekennzeichnet, daß ein Oxid oder Mischoxid des Ca und/oder Mg verwendet wird.

11. Verfahren nach Anspruch 9
dadurch gekennzeichnet,daß ein Oxid oder Mischoxid des Co und/oder Ni verwendet wird.

12. Verfahren nach Anspruch 9,
dadurch gekennzeichnet, daß Co₃O₄ eingesetzt wird.

13. Verfahren nach einem oder mehreren der vorhergehenden Ansprüche,
dadurch gekennzeichnet, daß ein Katalysator eingesetzt wird, der mit einem Metall aus der Gruppe VIII des Periodensystems dotiert ist.

14. Verfahren nach Anspruch 13,
dadurch gekennzeichnet, daß ein mit Ni und/oder Co dotierter Katalysator verwendet wird.

15. Verfahren nach Anspruch 13 oder Anspruch 14,
dadurch gekennzeichnet, daß das Dotierungsmetall in einer Menge von 0,1 - 60 Gew.-% (wt/wt) bezogen auf das Oxid oder Mischoxid eingesetzt wird.

16. Verfahren nach einem oder mehreren der Ansprüche 13 - 15,
dadurch gekennzeichnet, daß ein mit Ni und/oder Co dotiertes γ-Al₂O₃ oder Co₃O₄ eingesetzt wird.

17. Verfahren gemäß Anspruch 3,
dadurch gekennzeichnet, daß man als Katalysator Acetylacetonate von Übergangsmetallen der Gruppen IV_{b}, V_{b}, VI_{b} und VIII sowie von Aluminium einsetzt.

18. Verfahren gemäße Anspruch 3,
dadurch gekennzeichnet, daß man Brönstedtsäuren mit einem pK-Wert von 2-5 einsetzt.

19. Verfahren gemäß Anspruch 8,
**dadurch gekennzeichnet,**
daß man ein Carbonat oder ein Halogenid der genannten Elemente einsetzt.

20. Verfahren gemäß Anspruch 19,
**dadurch gekennzeichnet,**
daß man ein Cobalt- oder Nickelcarbonat, gegebenenfalls in der Hydratform einsetzt.

21. Verfahren nach einem der vorhergehenden Ansprüche,
dadurch gekennzeichnet, daß der Katalysator in einem Verhältnis von zwischen 0,01 und 50 Gew.-% (wt/wt) bezogen auf α-Isophoron eingesetzt wird.

22. Verfahren nach Anspruch 15,
dadurch gekennzeichnet, daß der Katalysator in einem Verhältnis von zwischen 0,2 und 10 Gew.-% (wt/wt) bezogen auf α-Isophoron eingesetzt wird.

23. Verfahren nach Anspruch 16,
dadurch gekennzeichnet, daß der Katalysator in einem Verhältnis von zwischen 0,5 und 5 Gew.-% (wt/wt) bezogen auf α-Isophoron eingesetzt wird.

24. Verfahren gemäß Anspruch 3,
dadurch gekennzeichnet, daß der homogene Katalysator in einem Verhältnis von 0,1 bis 1 Gew.-%, bezogen auf das α-Isophoron, eingesetzt wird.

25. Verfahren nach einem oder mehreren der vorhergehenden Ansprüche,
dadurch gekennzeichnet, daß bei Temperaturen zwischen 100 und <300 °C, bevorzugt 150 - 260 °C, isomerisiert wird, wobei der Druck so eingestellt wird, daß die Reaktion in der Flüssigphase abläuft.

26. Verfahren nach Anspruch 25,
dadurch gekennzeichnet, daß die Isomerisierung bei Normaldruck (etw. 1 x 10⁵ Pa) und der Siedetemperatur des α-Isophorons abläuft.

27. Verfahren nach Anspruch 7,
dadurch gekennzeichnet, daß man die Isomerisierung bei einer Temperatur von 130 bis 250 ° C und einem Druck von 1000 bis 1,5 x 10⁵ Pa durchführt, kontinuierlich Reaktionsgemisch abzieht und dieses bei einem Druck von 100 bis 3 x 10⁴ Pa destilliert und den Destillationssumpf gegebenenfalls in die Isomerisierung zurückführt.

## Claims

1. Process for the preparation of 3,5,5-trimethylcyclohexa-3-en-1-one (β-isophorone) by the catalysed isomerisation of 3,5,5-trimethylcyclohexa-2-en-1-one (α-isophorone) in the liquid phase,
characterised in that there is drawn off from the reaction mixture during the isomerisation, by distillation, a mixture consisting of from 0.5 to 75 wt.% β-isophorone, and the β-isophorone is isolated from that mixture by vacuum distillation (pure distillation).

2. Process according to claim 1,
characterised in that the isomerisation takes place with heterogeneous catalysis.

3. Process according to claim 1,
characterised in that the isomerisation takes place with homogeneous catalysis.

4. Process according to claims 1 to 3,
characterised in that the mixture consists of from 0.5 to 60 wt.% β-isophorone.

5. Process according to claims 1 to 3,
characterised in that the mixture consists of from 0.5 to 40 wt.% β-isophorone.

6. Process according to claims 1 to 5,
characterised in that the residues from the pure distillation are fed back into the isomerisation stage.

7. Process according to claims 1 to 6,
characterised in that the process is carried out continuously.

8. Process according to claims 1, 2, 4 to 7,
characterised in that there is used as the catalyst an oxide or mixed oxide of an element of groups IIₐ, VIII, I_{b}, IIIₐ and Vₐ of the periodic system, or salts of the mentioned elements that are insoluble under test conditions, or SiO₂, or silica gel, and the isomerisation is optionally carried out with the addition of an organic base.

9. Process according to claims 1, 2, 4 to 7,
characterised in that an oxide or mixed oxide of an element of groups IIₐ and VIII of the periodic system is used.

10. Process according to claim 9,
characterised in that an oxide or mixed oxide of Ca and/or Mg is used.

11. Process according to claim 9,
characterised in that an oxide or mixed oxide of Co and/or Ni is used.

12. Process according to claim 9,
characterised in that Co₃O₄ is used.

13. Process according to one or more of the preceding claims,
characterised in that a catalyst doped with a metal of group VIII of the periodic system is used.

14. Process according to claim 13,
characterised in that a catalyst doped with Ni and/or Co is used.

15. Process according to claim 13 or claim 14,
characterised in that the doping metal is used in an amount of from 0.1 to 60 wt.% (wt./wt.), based on the oxide or mixed oxide.

16. Process according to one or more of claims 13 to 15,
characterised in that a γ-Al₂O₃ or Co₃O₄ doped with Ni and/or Co is used.

17. Process according to claim 3,
characterised in that acetyl acetonates of transition metals of groups IV_{b}, V_{b}, VI_{b} and VIII and of aluminium are used as the catalyst.

18. Process according to claim 3,
characterised in that Brönstedt acids having a pK value of from 2 to 5 are used.

19. Process according to claim 8,
characterised in that a carbonate or a halide of the mentioned elements is used.

20. Process according to claim 19,
characterised in that a cobalt or nickel carbonate, optionally in hydrate form, is used.

21. Process according to any one of the preceding claims,
characterised in that the catalyst is used in a ratio of from 0.01 to 50 wt.% (wt./wt.), based on α-isophorone.

22. Process according to claim 15,
characterised in that the catalyst is used in a ratio of from 0.2 to 10 wt.% (wt./wt.), based on α-isophorone.

23. Process according to claim 16,
characterised in that the catalyst is used in a ratio of from 0.5 to 5 wt.% (wt./wt.), based on α-isophorone.

24. Process according to claim 3,
characterised in that the homogeneous catalyst is used in a ratio of from 0.1 to 1 wt.%, based on the α-isophorone.

25. Process according to one or more of the preceding claims,
characterised in that the isomerisation is carried out at temperatures of from 100 to <300°C, preferably from 150 to 260°C, the pressure being so adjusted that the reaction takes place in the liquid phase.

26. Process according to claim 25,
characterised in that the isomerisation takes place at normal pressure (approximately 1 x 10⁵ Pa) and at the boiling temperature of the α-isophorone.

27. Process according to claim 7,
characterised in that the isomerisation is carried out at a temperature of from 130 to 250°C and a pressure of from 1000 to 1.5 x 10⁵ Pa, reaction mixture is drawn off continuously and is distilled at a pressure of from 100 to 3 x 10⁴ Pa, and the distillation residues are optionally fed back into the isomerisation.

## Revendications

1. Procédé pour la préparation de 3,5,5-triméthylcyclohexa-3-ène-1-one (β-isophorone) par l'isomérisation catalysée de 3,5,5-triméthylcyclohexa-2-ène-1-one (α-isophorone) en phase liquide, caractérisé en ce qu'on retire par distillation du mélange réactionnel, pendant l'isomérisation, un mélange constitué par 0,5 à 75 % en poids de β-isophorone et dont on isole la β-isophorone par distillation sous vide (distillation de la substance pure).

2. Procédé selon la revendication 1, caractérisé en ce que l'isomérisation se déroule par catalyse en phase hétérogène.

3. Procédé selon la revendication 1, caractérisé en ce que l'isomérisation se déroule par catalyse en phase homogène.

4. Procédé selon les revendications 1 à 3, caractérisé en ce que le mélange est constitué de 0,5 à 60 % en poids par de la β-isophorone.

5. Procédé selon les revendications 1 à 3, caractérisé en ce que le mélange est constitué de 0,5 à 40 % en poids par de la β-isophorone.

6. Procédé selon les revendications 1 à 5, caractérisé en ce qu'on recycle le résidu de la distillation de la substance pure dans l'étape d'isomérisation.

7. Procédé selon les revendications 1 à 6, caractérisé en ce que le procédé est réalisé en continu.

8. Procédé selon les revendications 1, 2, 4 à 7, caractérisé en ce qu'on utilise comme catalyseur, un oxyde ou un oxyde mixte d'un élément des groupes IIₐ, VIII, I_{b}, IIIₐ et Vₐ du système périodique ou; sous des conditions opératoires d'essai, des sels insolubles des éléments mentionnés ou du SiO₂ ou du silicagel et en ce que l'isomérisation est, le cas échéant, réalisée en ajoutant une base organique.

9. Procédé selon les revendications 1, 2, 4 à 7, caractérisé en ce qu'on utilise oxyde ou un oxyde mixte d'un élément des groupes IIₐ et VIII du système périodique.

10. Procédé selon la revendication 9, caractérisé en ce qu'on utilise un oxyde ou un oxyde mixte de Ca et/ou de Mg.

11. Procédé selon la revendication 9, caractérisé en ce qu'on utilise un oxyde ou un oxyde mixte de Co et/ou de Ni.

12. Procédé selon la revendication 9, caractérisé en ce qu'on utilise du Co₃O₄.

13. Procédé selon l'une ou plusieurs des revendications précédentes, caractérisé en ce qu'on utilise un catalyseur dopé par un métal du groupe VIII du système périodique.

14. Procédé selon la revendication 13, caractérisé en ce qu'on utilise un catalyseur dopé avec du Ni et/ou du Co.

15. Procédé selon la revendication 13 ou la revendication 14, caractérisé en ce qu'on met en oeuvre le métal de dopage en une quantité de 0,1 à 60 % en poids (P/P) par rapport à l'oxyde ou à l'oxyde mixte.

16. Procédé selon l'une ou plusieurs des revendications 13 à 15, caractérisé en ce qu'on utilise du γ-Al₂O₃ ou du Co₃O₄ dopé avec du Ni et/ou du Co.

17. Procédé selon la revendication 3, caractérisé en ce qu'on utilise comme catalyseur de l'acétylacétonate de métaux de transition des groupes IV_{b}, V_{b}, VI_{b} et VIII ainsi que d'aluminium.

18. Procédé selon la revendication 3, caractérisé en ce qu'on utilise des acides de Brônstedt présentant un pK de 2 à 5.

19. Procédé selon la revendication 8, caractérisé en ce qu'on utilise un carbonate ou un halogénure des éléments mentionnés.

20. Procédé selon la revendication 19, caractérisé en ce qu'on utilise un carbonate de cobalt ou de nickel, le cas échéant sous forme hydratée.

21. Procédé selon l'une quelconque des revendications précédentes, caractérisé en ce que le catalyseur est mis en oeuvre dans un rapport compris entre 0,01 et 50 % en poids (P/P) par rapport à l'α-isophorone.

22. Procédé selon la revendication 15, caractérisé en ce que le catalyseur est mis en oeuvre dans un rapport compris entre 0,2 et 10 % en poids (P/P) par rapport à l'α-isophorone.

23. Procédé selon la revendication 16, caractérisé en ce que le catalyseur est mis en oeuvre dans un rapport compris entre 0,5 et 5 % en poids (P/P) par rapport à l'α-isophorone.

24. Procédé selon la revendication 3, caractérisé en ce que le catalyseur en phase homogène est mis en oeuvre dans un rapport de 0,1 à 1 % en poids par rapport à l'α-isophorone.

25. Procédé selon l'une ou plusieurs des revendications précédentes, caractérisé en ce que l'isomérisation est réalisée à des températures entre 100 et <300 °C, de préférence entre 150 et 260 °C, la pression étant réglée de telle manière que la réaction se déroule en phase liquide.

26. Procédé selon la revendication 25, caractérisé en ce que l'isomérisation se déroule sous pression normale (environ 1 x 10⁵ Pa) et à la température d'ébullition de l'α-isophorone.

27. Procédé selon la revendication 7, caractérisé en ce que l'isomérisation est réalisée à une température de 130 à 250 °C et une pression de 1000 à 1,5 x 10⁵ Pa, en ce qu'on retire en continu le mélange réactionnel et en ce qu'on distille celui-ci à une pression de 100 à 3 x 10⁴ Pa et en ce qu'on recycle, le cas échéant, le résidu de distillation dans l'isomérisation.
